# EUROPEAN PATENT SPECIFICATION

(11) **EP 3 735 406 B1**
(45) Date of publication and mention of the grant of the patent: **11.05.2022**
(21) Application number: 18702558.0
(22) Date of filing: 02.01.2018
(51) Int. Cl.: C07D 215/26

(54) **A PROCESS FOR PREPARATION OF 5-(2-(SUBSTITUTED-AMINO)-1-HYDROXYETHYL)-8-(SUBSTITUTED-OXY) QUINOLIN-2(1H)-ONE**
VERFAHREN ZUR HERSTELLUNG VON 5-(2-(SUBSTITUIERTEM-AMINO)-1-HYDROXYETHYL)-8-(SUBSTITUIERTEM-OXY)-CHINOLIN-2(1H)-ON
PROCÉDÉ DE PRÉPARATION DE 5-(1-HYDROXYÉTHYLE À SUBSTITUTION AMINO EN POSITION 2)-QUINOLÉINE-2(1H)-ONE À SUBSTITUTION OXY EN POSITION 8

(43) Date of publication of application: 11.11.2020
(73) Proprietor: Deva Holding Anonim Sirketi, 34303 Istanbul (TR)
(72) Inventor: HAAS, Philipp Daniel, 34303 Istanbul (TR); STECKEL, Andreas Hartwig, 34303 Istanbul (TR); KARLIGA, Bekir, 34303 Istanbul (TR); BELLUR ATICI, Esen, 34303 Istanbul (TR); YILMAZ, Halil, 34303 Istanbul (TR)
(86) International application number: PCT/IB2018/050005
(87) International publication number: WO 2019/135101

(56) References cited:
- WO-A2-2016/027283

## Description

### Technical Field

The present invention relates to a process for the preparation of indacaterol intermediate with higher yield and enantiopurity. The present invention relates to an improved method for synthesis of intermediates which are useful for the preparation of Indacaterol or pharmaceutically acceptable salts and are represented by the structure of formula A.

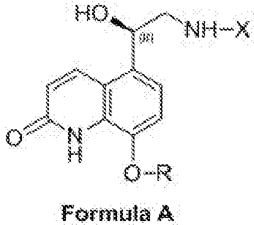

### Background Art

Indacaterol maleate is a selective beta2-adrenergic agonist. It is designed chemically as 5-{(1R)-2-[(5, 6-diethyl-2, 3-dihydro-1H-inden-2-yl) amino]-1-hydroxyethyl}-8-hydroxy-2(1H)-quinolinone maleate (Fig. 1). Indacaterol is also a chiral molecule but only the pure (R)-enantiomer is dispensed.

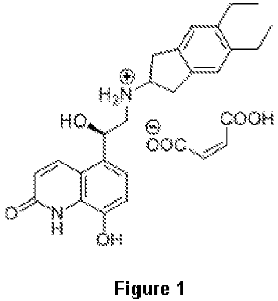

Indacaterol as a long-acting beta-2 adrenergic agonist (LABA) causes bronchodilator by relaxing the smooth muscle in the airway and is intended for long-term maintenance treatment of inflammatory or obstructive airways disease such as chronic obstructive pulmonary disease (COPD). Indacaterol is marketed by Novartis under the trade name Arcapta Neohaler^{®} in US and Onbrez^{®} in Europe.

Various synthetic processes for preparation of Indacaterol, its intermediates and related compounds are known in the art.

Indacaterol was firstly disclosed in WO00/75114. The synthesis of indacaterol maleate was described in WO00/75114 as depicted in Scheme 1.

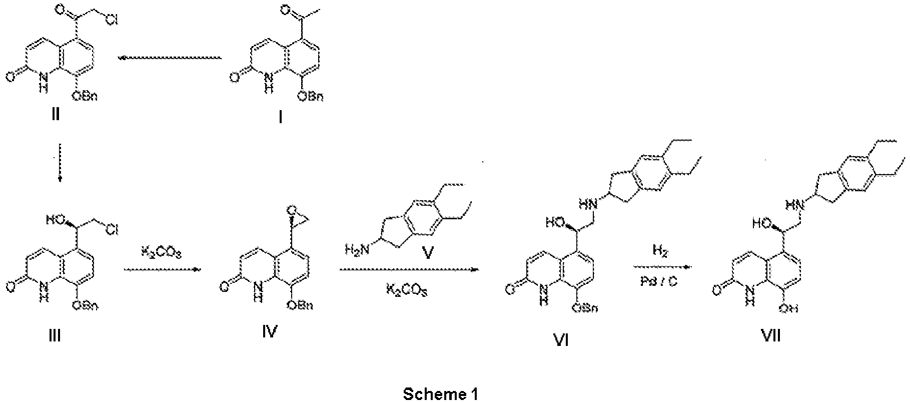

The process depicted in scheme 1 involves the reaction of an epoxide, 8- substituted oxy-5-(R)-oxiranyl-(IH)-quinolin-2-one (IV) with an amine intermediate, 2-amino- (5,6-diethyl)-indan (V) to give an intermediate 5-[(R)-2-(5,6-diethyl-indan-2- ylamino)-I-hydroxy-ethyl]-8-substituted oxy-(IH)-quinolin-2-one (VI). The compound of formula (VI) was isolated by flash chromatography, and then it was converted into indacaterol maleate after removing the benzyl group and treating the resulting indacaterol base with maleic acid.

One of the drawbacks of the process depicted in Scheme 1 involves an epoxide ring opening reaction. This epoxide ring opening is not regiospecific. Thereby along with 5-[(R)-2-(5, 6- diethyl-indan-2-ylamino) - 1 -hydroxyl-ethyl]-8-substituted oxy-(1 H)-quinolin-2-one, below its regioisomeric and dimeric side-products are being produced as impurities.

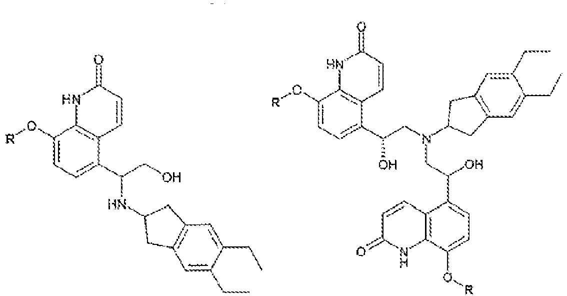

The separation of desired compound from isomeric impurities is of great importance. Thus, 5-[(R)-2-(5, 6- diethyl-indan-2-ylamino) - 1 -hydroxyl-ethyl]-8-substituted oxy-(1 H)-quinolin-2-one should be isolated from its dimeric and regioisomeric side-products. In the above process, 5-[(R)-2-(5, 6- diethyl-indan-2-ylamino) - 1 -hydroxylethyl]-8-substituted oxy-(1 H)-quinolin-2-one is isolated and purified by using column chromatography. This method is tedious and requires large amounts of solvents and it is not preferable for industrial manufacturing.

WO 2004/076422 discloses a process that avoids the column purification by the formation of acid addition salts of intermediate. The mentioned process involves the formation of undesired regioisomer and dimer side products during the reaction of 8-benzyloxy-5-(R)-oxiranyl-(1H)-quinolin-2-one with 2-amino-5,6-diethylindane (Scheme 2).

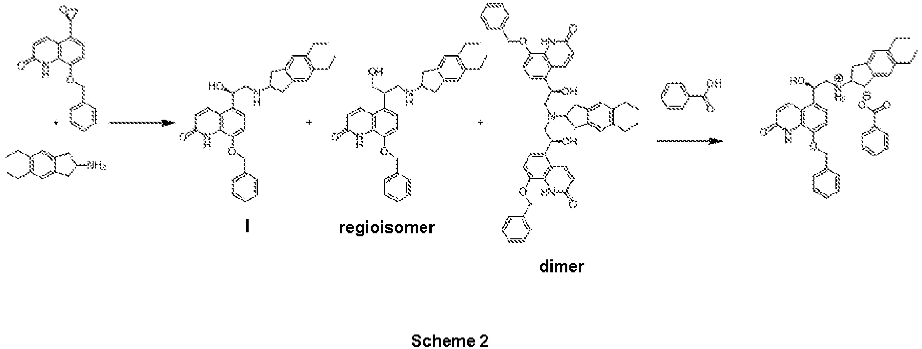

8-(benzyloxy)-5-(2-((5,6-diethyl-2,3-dihydro-1H-inden-2-yl)amino)-1-hydroxyethyl)quinolin-2(1H)-one shown as compound of formula I, is very important intermediate for the preparation of indacaterol maleate. Compound of formula (I) is purified from the undesired side products via conversion to its benzoic acid salt. However, the yield for benzoic acid salt of preferable regioisomer compound of formula (I) is low, about 60% and having 96% enantiomeric purity. Then, it is recrystallized, reduced with hydrogen, converted to indacaterol maleate.

The route of synthesis disclosed in WO2004/076422 and WO00/75114 comprise an epoxide starting material to obtain 5-[(R)-2-(5, 6- diethyl-indan-2-ylamino) - 1 - hydroxyl-ethyl]-8-substituted oxy-(1 H)-quinolin-2-one. According to examples of said patents, the epoxide ring opening reactions occur at high temperatures, about 110 °C for 15 hours. However, energy and time consuming reactions at high temperatures for long hours are not favourable in economic aspect.

WO2014044566A1 discloses a process for preparing indacaterol or a pharmaceutically acceptable salt thereof. In this process 2-amino-5, 6-diethylindan, compound of formula II, reacts with the compound of formula I.

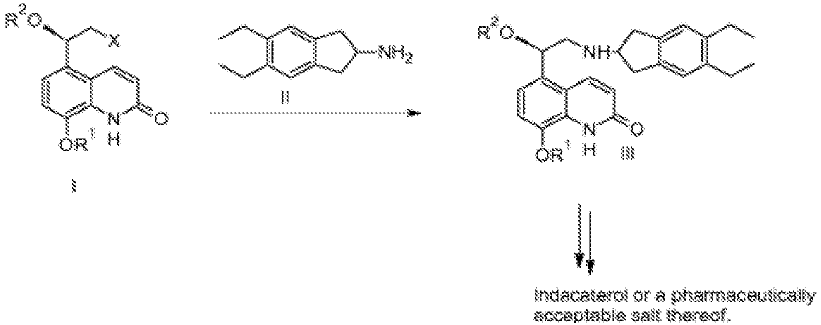

The compound of formula I is obtained from the corresponding hydroxyl-unprotected compound of formula VI:

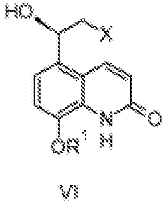

The hydroxyl group of compound of formula VI should be protected before the reaction with 2-amino-5, 6-diethylindan, compound of formula II. The compound of formula I is obtained by protection hydroxyl group of compound of formula VI. After that, the reaction of compound of formula I with 2-amino-5, 6-diethylindan gives compound of formula III, hydroxyl group of compound of formula III is removed by deprotection process.

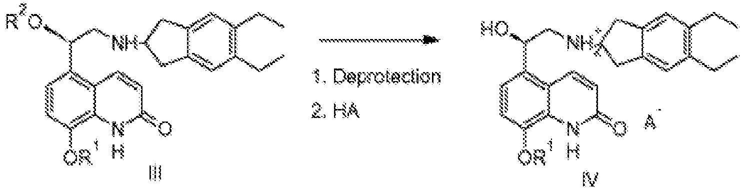

However, additional protection and deprotection steps in the above mentioned process may raise the need of extra steps for the purification of undesired impurities. Furthermore, increasing the process complexity not only results in the loss of efficiency and a higher operation cost, but also requires higher initial investment due to the extensive need in solvents and related equipments to handle them.

WO2016027283A2 discloses a new process for synthesis of indacaterol. This process comprises of two different intermediates (Formula 2 and Formula 3). L is a leaving group and R' is hydrogen or amino protecting group.

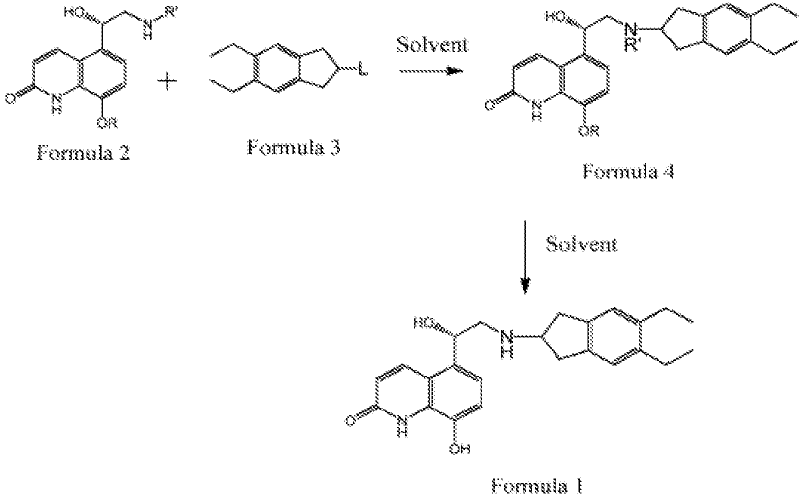

According to this process; Formula 8 is obtained firstly by treating compound of Formula 6 with compound of Formula 7 at the temperature range between 0 °C and 120 ° C. The step of reaction is carried out in the presence of solvent and in the presence of base. Formula 7 is a cyclic imide such as phthalimide or succinimide.

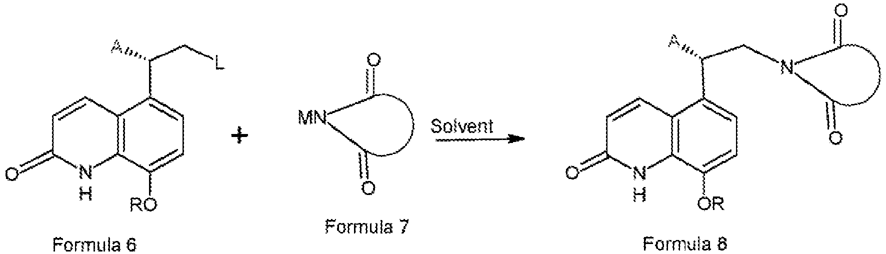

Compound of Formula 2 is prepared from compound of Formula 8 which is treated with a reducing agent in the presence of catalyst and followed by treatment with an amine deprotecting group.

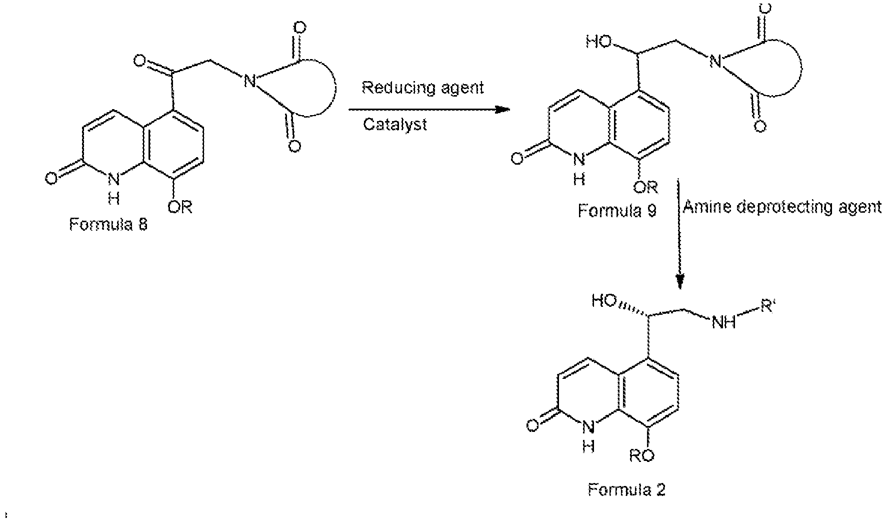

If Formula 8 have an OH group instead of =O, Formula 8 is treated with an amine deprotecting group to obtain Formula 2.

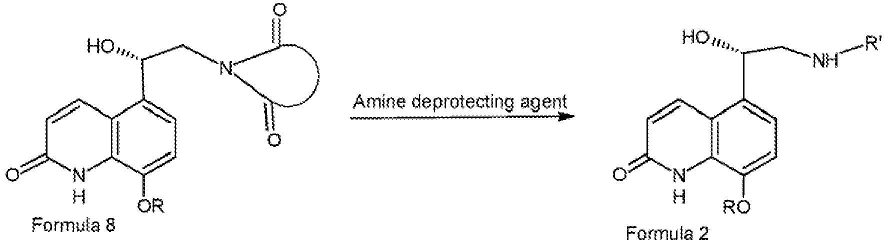

If Formula 6 consist of A is =O, L is H and R is an OH protecting group which on reaction with an oxidizing agent and arylalkylamines, respectively. Finally, Formula 6b is reducted with a reducing agent and Formula 2 is obtained.

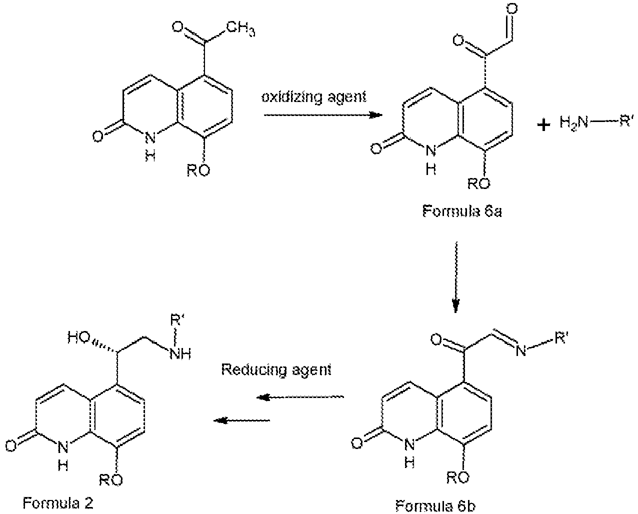

According to examples of Method A and Method B which are disclosed in WO2016027283A2, the compound of formula 2 is obtained with high yields. However the patent is silent about the enantiomeric purity for said compound.

Therefore, based on the drawbacks mentioned in all the prior arts, there still exists a need to develop an improved process for preparing intermediates of indacaterol and pharmaceutically acceptable salts thereof. The said process should be simple, cost-effective and suitable for large scale industrial operation of indacaterol or pharmaceutically acceptable salts thereof.

By decreasing the operation and production cost of the intermediates by utilizing more effective methods for preparation, the cost of the final product is also reduced, thus enabling the patients easier access to higher quality medicine.

### Summary of the invention

This invention provides a novel process with lower cost for the preparation of novel intermediates in the synthesis of Indacaterol or pharmaceutically acceptable salts thereof. The present invention provides excellent yields and purity, while not requiring the purification from the synthesis regioisomeric and dimeric side-products.

### Technical Problem

Active pharmaceutical ingredients (APIs) are individual components or mixture of components that are used as a part of a finished pharmaceutical drug or medical product, where they provide the pharmacological activity. Therefore they are the first step of the pharmaceutical development and the improvement of pharmaceutical industry is strongly related to the changes and to the progress in the API production methods and API quality.

The quality of active pharmaceutical ingredients in a drug has a direct effect on the safety and efficiency of that drug, which is the main reason behind the strict quality and purity standards applied on these APIs. Poorly manufactured and contaminated active ingredients may have a high risk of affecting the general health of the patient negatively rather than treating the disease.

The research and development projects in the pharmaceutical industry mainly aim to investigate different possible synthetic routes, key intermediates, reaction steps, polymorphism, impurity profile, particle size and shape to produce these APIs with higher efficiency and lesser initial investment.

Technical challenges involve a multitude of issues designed to improve yield, purity, stereoselectivity, process conditions (i.e., temperature and pressure), scalability, and production economics.

Most APIs can be synthesized using any of several alternative pathways.

By using various synthetic pathways, it is possible to lower the production costs and simplify the process; therefore it has paramount importance to choose the right one for the general efficiency and success of the operation.

In the pharmaceutical industry; the synthetic route of API that uses low-cost starting materials is one of the critical parameters in process development.

Another critical parameter in process development is the purification step, which is time consuming and expensive due to the complex nature of the APIs and their impurities.

Certain synthetic routes require less purification due to the relatively less sensitivity of the API. A wide range of APIs and their production methods do not require the use of toxic solvents, which are hard to dispose of and therefore more expensive to use. Any of these can provide an economical advantage.

The currently used API purification are among others distillation, adsorption-desorption, solvent extraction, fractional crystallisation and chromatography.

Chromatography has its own limitations on commercial scale; since it is an expensive and time consuming operation if applied to industrial scale. Furthermore, this technique also consumes a lot of solvents, which are hazardous for environment. Purification by using column chromatography is not preferable method for industrial scale productions.

There is no doubt that the choice of starting materials and the design of the synthetic route are critical, particularly the number of synthetic steps and the amount of yield in the synthesis.

Reducing the number of process steps to produce an active pharmaceutical ingredient enables circumventing several purification procedures, reducing the length of process time, and also minimizing chemical waste to be disposed. Therefore it is very critical to have the simplest process possible to obtain a high quality product with high performance/cost ratio.

In any synthetically procedure there are many factors; such as, temperature, time, pressure, reagents, rate of addition, catalyst, solvent, concentration and pH that will have an influence on the overall yield, purity and selectivity.

A general rule for the evaluation of reaction temperature is that increasing the reaction temperature causes an increase in the reaction rate. However, this will also increase the potential for by-product formation, which could adversely impact both product yield and quality. Thus, it is preferable to optimize the process temperature range to give the maximum conversion rate of the starting materials to the end product in the shortest time, with the least amount of impurities.

Due to the extensive use of indacaterol with high purity and yield in many pharmaceuticals, the research projects aiming for a better process efficiency and a higher purity end product, are still continuing on increasingly. That being said; the purity and yield of the product can also be increased by purification processes such as re-crystallization, which on the other hand will raise the complexity of the process, thus increasing the cost and lowering the efficiency.

### Solution to Problem

For deficiencies in the prior art, our inventors developed an advanced process for the synthesis of intermediate compounds to be used in the preparation of indacaterol. Compared with the processes in the prior art, new method of the present invention provides a process involving the use of cheap raw starting materials, mild reaction conditions, sufficient selectivity, purification without column chromatography and suitable for industrialization.

Our inventors tried to explore the possibility of improving the yield and reducing the cost for manufacturing of indacaterol. For this purpose they developed a process for preparation of compound of formula A which is an intermediate involved in the indacaterol synthesis. The new synthesis of intermediates shown as compound of formula A provides a significant economic benefit compared to the available alternative synthetic pathways of the other intermediates in the indacaterol synthesis.

The present invention relates to an improved method for synthesis of intermediates which are useful for the preparation of Indacaterol or pharmaceutically acceptable salts and are represented by the structure of formula A.

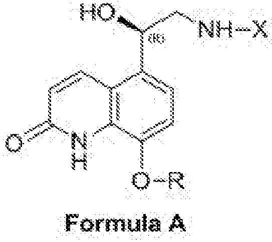

The new synthesis of compound of formula A provides in high selectivity and yield, and minimizing or eliminating the formation of regioisomers.

The new synthesis of compound of formula A also provides a process which is suitable for large scale commercial production.

A first aspect of the present invention relates to a process for preparing the compound of formula H, wherein the process comprises of reacting 8-hydroxy-2-oxo-1,2-dihydroquinoline-5-carbaldehyde(compound of formula F) with a nitromethane in the presence of solvent and catalyst or a base to obtain Formula H.

A second aspect of the present invention relates to a process for preparing of the compound of formula Z, wherein the compound of formula H reacted with a reducing agent in the presence of catalyst to form compound of Formula Y followed by treatment with an amine protected group to obtain Formula Z.

The compound of formula A is prepared from the compound of Formula Z using by various hydroxyl protecting group

The present invention is described in Scheme 3.

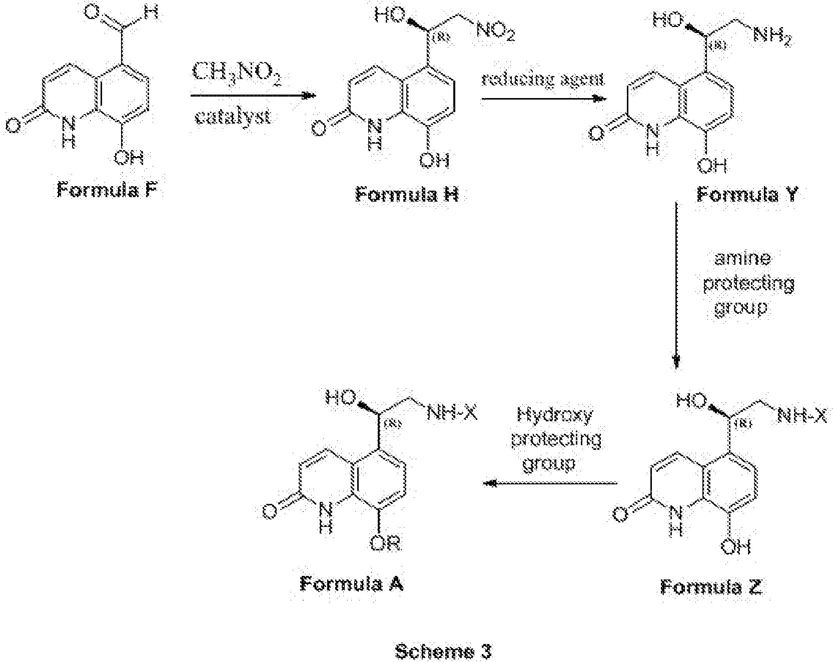

When compared to prior art, the advantage of this process for the synthesis of indacaterol intermediate is the use of an easily accessible starting material. Use of 8-hydroxy-2-oxo-1, 2-dihydroquinoline-5-carbaldehyde as commercially readily available and cheap starting material contributes to reducing the manufacturing cost.

The newly developed synthesis presents high yields in all steps of the synthesis. The synthesis is also beneficial from the economical point of view as the uses of the expensive agents are eliminated.

Our inventors with this simple and cost-effective preparation method for obtaining indacaterol intermediate would be also contributed to reduce the production cost of indacaterol and being easily available to patients.

The present invention process for the indacaterol intermediate comprises the step of;
i) reacting the 8-hydroxy-2-oxo-1,2-dihydroquinoline-5-carbaldehyde 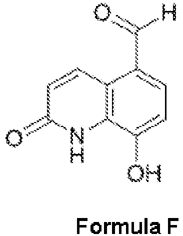 with nitromethane to obtain compound of formula H in the presence of a base, catalyst and a suitable solvent, 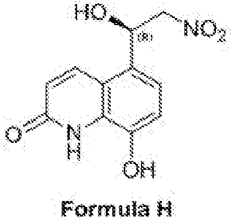
ii) treating the compound of formula H with reducing agent to obtain compound of formula Y 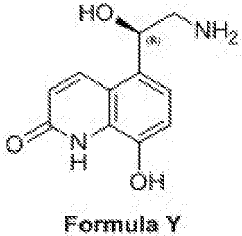
iii) treating the compound of formula Y with amine protecting group to obtain compound of formula Z 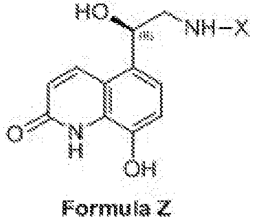 where X is an amine protecting group
iv) reacting the compound of formula Z with hydroxyl protecting group to obtain compound of formula A 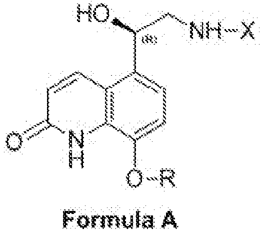 where R is a hydroxyl protecting group, X is an amine protecting group.

Use of compound of formula A comprising protected amine group in the preparation of indacaterol also avoids some unwanted side-products may be caused by the use of primary amine during the synthesis of indacaterol.

The base employed in the step (i) is selected from the group of organic base or inorganic base, wherein organic base is selected from cyclic or acyclic amines, while bases diethylamine, triethylamine and diisopropylethylamine may be operated in alcoholic solvents. Inorganic base may be selected from the groups of alkali metal hydroxide like sodium hydroxide, calcium hydroxide, potassium hydroxide or alkali metal carbonate like sodium carbonate, potassium carbonate, while base is alkali metal salt the reaction may be operated in aqueous solvent systems.

The suitable polar and non polar organic solvents or solventless conditions employed in step (i).

Examples for suitable non polar organic solvents are may be used is step (i) include; pentane, hexane, cyclohexane, toluene, chloroform, diethyl ether and 1,4-dioxane.

Examples for suitable polar organic solvents may be selected from the group of polar aprotic solvents, such as, acetone, ethyl methyl ketone, methyl isobutyl ketone, acetonitrile, ethyl acetate, N, N-Dimethylformamide (DMF), Dimethylsulfoxide (DMSO), dichloromethane and mixtures thereof.

Examples for suitable polar organic solvents may be selected from the group of polar protic solvents, such as, ammonia, methanol, ethanol, n-propanol, n-butanol, t-butanol, isopropyl alcohol (IPA), acetic acid, Tetrahydrofuran(THF) and water.

Solventless condition employed in step (i) is selected large excess of nitromethane.

When chosen solventless condition employed in step (i), the molar ratio between 8-hydroxy-2-oxo-1,2-dihydroquinoline-5-carbaldehyde and nitromethane is about 1:2. Preferably the nitromethane is used in excess with respect to 8-hydroxy-2-oxo-1,2-dihydroquinoline-5-carbaldehyde, e.g. a preferred molar ratio is 1:4 and advantageously 1:10.

Many different catalysts employed in step (i) may be selected from the group of ligands, complexes or guanidine-containing organocatalysts.

Examples for suitable catalysts in step (i) include but are not limited to complexes. Suitable complexes are selected from the group of La-(R)-BINOL complex, Ln-Li-(R)-BINOL complex, (S)-(-)-1,1'-Bis(2-naphthol)-NdCl₃•6H₂O complex, Cu-bis(oxazolidine) complex like , (+)-2, 2'-Isopropylidenebis[(4R)-4-phenyl-2-oxazoline]-Cu(OAc)₂.H₂O and (+)-2, 2'-Isopropylidenebis[(4R)-4-phenyl-2-oxazoline]- Copper(II) trifluoromethanesulfonate.

Guanidine-containing organocatalysts such as 1, 1, 3, 3-tetramethylguanidine (TMG) is preferably employed in step (i) in different solvent such as THF or diethyl ether.

When a catalyst system was employed in the stereoselective preparation of Formula H, the reaction afforded in 94-98% enantiomeric purity and 70-90% chemical yields.

A suitable temperature range for performing reaction in step (i) is 20 to 60°C.

The reduction in step (ii) may be carried out with catalytic hydrogenation. Preferred catalysts include palladium, palladium hydroxide, palladium on activated carbon, palladium on alumina, palladium on carbon powder, platinum, platinum on activated carbon and Raney^{™} nickel. A combination of catalysts may also be used. Most preferably, the catalyst is palladium on activated carbon.

R is a hydroxyl protecting group selected from lower alkyl groups (e.g t-butyl), lower alkenyl groups (e.g allyl); lower alkanoyl groups (e.g acetyl); lower alkoxycarbonyl groups (e.g t-butoxycarbonyl); lower alkenyloxycarbonyl groups (eg allyloxycarbonyl); aryl lower alkoxycarbonyl groups (e.g benzoyloxycarbonyl, p-methoxybenzyloxycarbonyl, o-nitrobenzyloxycarbonyl, p-nitrobenzyloxycarbonyl); tri lower alkylsilyl (e.g trimethylsilyl, t-butyldimethylsilyl) and aryl lower alkyl (eg benzyl) groups.

X is an amine protecting group selected from aralkyl groups (eg: benzyl and substituted benzyl (eg: p-methoxybenzyl, nitrobenzyl and 2,4-dimethoxybenzyl); lower alkoxycarbonyl (eg t-butoxycarbonyl); lower alkenyloxycarbonyl (eg: allyloxycarbonyl); aryl lower alkoxycarbonyl groups (eg: benzyloxycarbonyl, p-methoxybenzyloxycarbonyl, o-nitrobenzyloxycarbonyl, p-nitrobenzyloxycarbonyl); trialkylsilyl (eg: trimethylsilyl and t-butyldimethylsilyl); alkylidene (eg: methylidene); benzylidene and substituted benzylidene groups; carbamates (eg tert-butyl carbamate, benzyl carbamate) and amides (eg: tosylamide, phthalimide, acetamide).

A third aspect of the present invention relates to a method of applying the compound of formula A in the synthesis of indacaterol. The method is simple, low cost and suitable for industrial scale manufacturing.

Synthesis of indacaterol involving compound of formula A is described in Scheme 4.

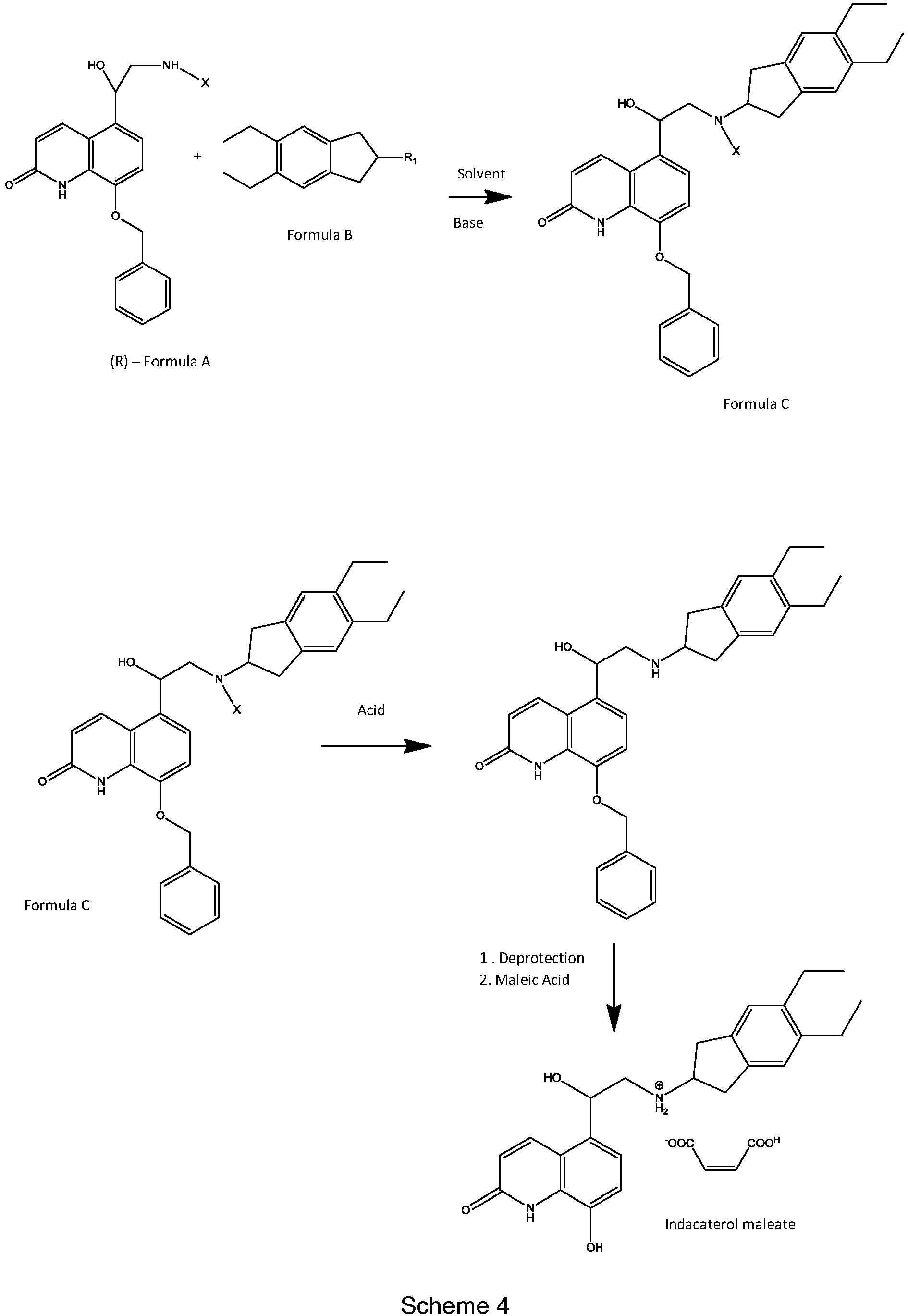

Condensation of compound of formula B with compound of formula A in the presence of a base and catalyst yields compound of formula C, which is subjected to deprotection with H₂ over Pd/C, Methanol. After deprotection step, indacaterol maleate is obtained via addition of maleic acid.

The following examples are provided to enable one skilled in the art to practise the invention and are merely illustrative of the invention. The examples should not be read as limiting the scope of the invention.

### Examples

### Example-1

### Preparation of 8-hydroxy-5-(1-hydroxy-2-nitroethyl) quinolin-2(1H)-one, compound of formula H

A flask was charged with (+)-2, 2'-Isopropylidenebis[(4R)-4-phenyl-2-oxazoline] (0.06 eq), Copper(II) trifluoromethanesulfonate (0.05 eq) and EtOH in a nitrogen atmosphere. The mixture was stirred for 1h and obtained blue solution. Nitromethane (10 eq) and 8-hydroxy-2-oxo-1, 2-dihydroquinoline-5-carbaldehyde (1 eq) were added. The reaction was monitored with TLC, after completion of reaction water was added and then extracted with dichloromethane (50mLx2). The organic solvent was removed in vacuo to obtain oily product. (Yields 78%, enantiomeric purity 98%)

### Example-2

### Preparation of 8-hydroxy-5-(1-hydroxy-2-nitroethyl) quinolin-2(1H)-one, compound of formula H

A flask was charged with (+)-2, 2'-Isopropylidenebis[(4R)-4-phenyl-2-oxazoline] (0.06 eq), Cu(OAc)₂.H₂O (0.05 eq) and EtOH in a nitrogen atmosphere. The mixture was stirred for 1h and obtained blue solution. Nitromethane (10 eq) and 8-hydroxy-2-oxo-1,2-dihydroquinoline-5-carbaldehyde (1 eq) were added. The reaction was monitored with TLC, after completion of reaction water was added and then extracted with dichloromethane (50mLx2). The organic solvent was removed in vacuo to obtain oily product. (Yields 81%, enantiomeric purity 96%)

### Example-3

### Preparation of 8-hydroxy-5-(1-hydroxy-2-nitroethyl) quinolin-2(1H)-one, compound of formula H

A flask was charged with NdCl₃•6H₂O (0.02 eq), dry THF and (S)-(-)-1,1'-Bis(2-naphthol) (0.02 eq) in a nitrogen atmosphere. The mixture was stirred at 60 °C for 30 min then NaOtBu (0.12 eq) was added dropwise. The mixture was stirried for another 30 min at the same temperature. Afterwards the mixture was cooled to room temperature, and nitromethane (2 eq) was added. This mixture was stirred at room temperature for 3h. At the end of this time, the mixture was cooled to -40 °C and 8-hydroxy-2-oxo-1, 2-dihydroquinoline-5-carbaldehyde (1 eq) was added dropwise. After stirring the reaction mixture at the same temperature for 20 h, AcOH/THF was added and the resulting mixture was extracted with EtOAc. The organic solvent was removed in vacuo to obtain oily product. (Yields 79%, enantiomeric purity 98%)

### Example-4

### Preparation of 8-hydroxy-5-(2-amino-1-hydroxyethyl) quinolin-2(1H)-one, compound of formula Y

8-hydroxy-5-(1-hydroxy-2-nitroethyl) quinolin-2(1H)-one (1 eq) was dissolved in MeOH and 10% palladium on charcoal were added. The mixture was hydrogenated under an atmosphere of hydrogen until the conversion is complete. The reaction was monitored with TLC. The catalyst was filtered off and the solvent was removed in vacuo. To obtained product was used without further purification in the next step.

### Example-5

### Preparation of compound of formula Z

8-hydroxy-5-(2-amino-1-hydroxyethyl) quinolin-2(1H)-one (10 eq) was dissolved in 20 mL THF and di-tert-butyldicarbonate (12 eq mmol) was added. After several minutes, the mixture was concentrated under reduce pressure and the residue was partitioned between saturated sodium bicarbonate and ethyl acetate. The organic solvent washed with brine and dried over MgSO4. The product recrystallized with concentrated ethyl acetate.

### Example-6

### Preparation of compound of formula A

A flask was charged with formula Z (20 eq), potassium carbonate (26 eq) and Dimethylformamide. (20 mL). Benzyl bromide (20 eq) was added and the mixture was stirred at the room temperature for 3-4 hours. After completion of reaction saturated sodium chloride (375 mL) was added at the 0°C, and the mixture was stirred another 1 hour. The product was filtered and dried on vacuo. Yields 79%.

## Claims

1. A process for preparing for compound of formula A; 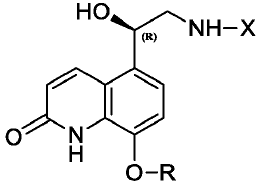
wherein X is an amine protecting group
R is a hydroxyl protecting group
comprising the steps of:
(i) treating the compound of formula F, 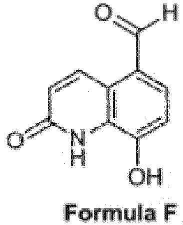 with nitromethane in the presence of solvent and strong base or catalyst to obtain compound of formula H; 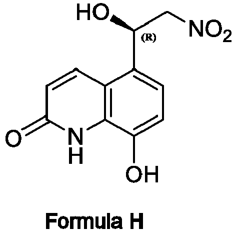
(ii) treating the compound of formula H with the reducing agent in the presence of solvent to obtain compound of formula Y; 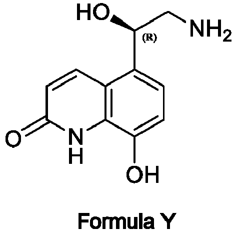
(iii) treating compound of formula Y with amine protecting group to obtain formula Z 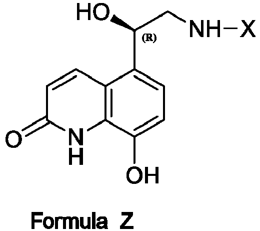
wherein X is an amine protecting group
R is a hydroxyl protecting group,
(iv) and then treating with hydroxyl protecting group to obtain compound of formula A.

2. The process according to claim 1, wherein the hydroxyl protecting group R of compound of formula A is benzyl.

3. The process according to claim 1, wherein the amine protecting group X is selected from the group comprising arylaklyl like; benzyl amine, triphenylmethylamine, carbamates like; tert-butyl carbamate, benzyl carbamate, amides like; tosylamide, phthalimide and acetamide.

4. The process according to claim 1, wherein the solvent is selected from the group of alcohols consisting methanol, ethanol and isopropyl alcohol.

5. The process according to claim 1, wherein the solvent is water miscible organic solvent such as THF.

6. The process according to claim 1, wherein said strong base is selected from the group consisting of organic base, inorganic base, alkali metal hydroxide and alkali carbonate.

7. The process according to claim 6, wherein said organic base is selected from the group consisting of diethylamine, triethylamine and diisopropylethylamine.

8. The process according to claim 6, wherein said alkali metal hydroxide is selected from the group consisting of sodium hydroxide, calcium hydroxide and potassium hydroxide.

9. The process according to claim 6, wherein said alkali carbonate is selected from the group consisting of sodium carbonate and potassium carbonate.

## Patentansprüche

1. Ein Verfahren zur Herstellung einer Verbindung der Formel A; 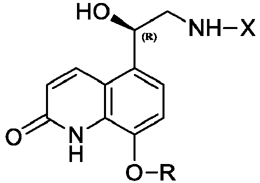
wobei X eine Aminschutzgruppe ist
R eine Hydroxylschutzgruppe ist
umfassend die folgenden Schritte:
(i) Behandeln der Verbindung der Formel F, 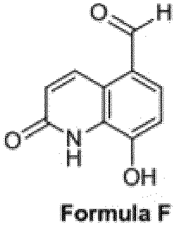 mit Nitromethan in Gegenwart von Lösungsmittel und einer starken Base oder eines Katalysators, um eine Verbindung der Formel H zu erhalten; 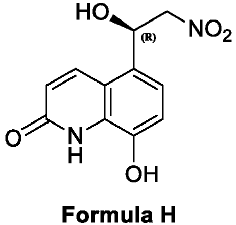
(ii) Behandeln der Verbindung der Formel H mit dem Reduktionsmittel in Gegenwart von Lösungsmittel, um eine Verbindung der Formel Y zu erhalten; 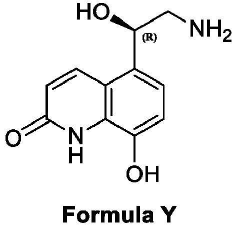
(iii) Behandeln der Verbindung der Formel Y mit der Aminschutzgruppe, um eine Verbindung der Formel Z zu erhalten; 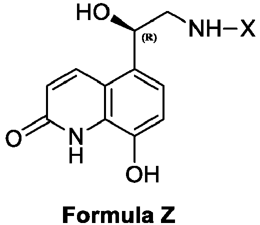
wobei X eine Aminschutzgruppe ist
R eine Hydroxylschutzgruppe ist,
(iv) und dann Behandeln mit der Hydroxylschutzgruppe, um eine Verbindung der Formel A zu erhalten.

2. Verfahren nach Anspruch 1, wobei Hydroxylschutzgruppe R der Verbindung der Formel A Benzyl ist.

3. Verfahren nach Anspruch 1, wobei die Aminschutzgruppe X ausgewählt ist aus der Gruppe umfassend Arylaklyl wie Benzylamin, Triphenylmethylamin, Carbamate wie tert-Butylcarbamat, Benzylcarbamat, Amide wie Tosylamid, Phthalimid und Acetamid.

4. Verfahren nach Anspruch 1, wobei das Lösungsmittel ausgewählt ist aus der Gruppe von Alkoholen bestehend aus Methanol, Ethanol und Isopropylalkohol.

5. Verfahren nach Anspruch 1, wobei das Lösungsmittel ein mit Wasser mischbares organisches Lösungsmittel wie THF ist.

6. Verfahren nach Anspruch 1, wobei die starke Base ausgewählt ist aus der Gruppe bestehend aus organischer Base, anorganischer Base, Alkalimetallhydroxid und Alkalicarbonat.

7. Verfahren nach Anspruch 6, wobei die organische Base ausgewählt ist aus der Gruppe bestehend aus Diethylamin, Triethylamin und Diisopropylethylamin.

8. Verfahren nach Anspruch 6, wobei das Alkalimetallhydroxid ausgewählt ist aus der Gruppe bestehend aus Natriumhydroxid, Calciumhydroxid und Kaliumhydroxid.

9. Verfahren nach Anspruch 6, wobei das Alkalicarbonat ausgewählt ist aus der Gruppe bestehend aus Natriumcarbonat und Kaliumcarbonat.

## Revendications

1. Procédé de préparation du composé de formule A 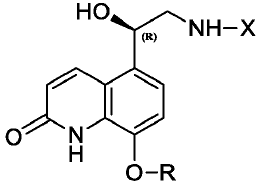
où X est un groupe protecteur d'amine
R est un groupe protecteur d'hydroxyle
comprenant les étapes de:
(i) le traitment le composé de formule F, 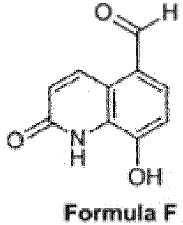 avec du nitrométhane en présence d'un solvant et d'une base forte ou d'un catalyseur pour obtenir un composé de formule H; 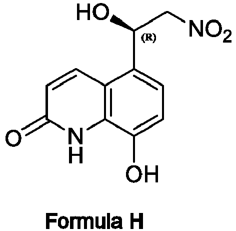
(ii) le traitement du composé de formule H avec l'agent réducteur en présence d'un solvant pour obtenir le composé de formule Y; 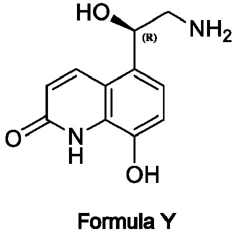
(iii) le traitment du composé de formule Y avec un groupe protecteur d'amine pour obtenir la formule Z. 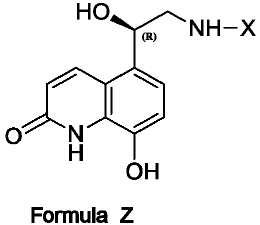
où X est un groupe protecteur d'amine
R est un groupe protecteur d'hydroxyle,
(iv) puis traitement avec un groupe protecteur d'hydroxyle pour obtenir le composé de formule A.

2. Procédé selon la revendication 1, dans le quel le groupe protecteur d'hydroxyle R du composé de formule A est le groupe benzyle.

3. Procédé selon la revendication 1, dans le quel le groupe protecteur d'amine X est choisi dans le groupe comprenant les arylaklyl similaires; la benzylamine, la triphénylméthylamine, les carbamates similaires; le carbamate de tert- butyle, le carbamate de benzyle, les amides similaires; tosylamide, phtalimide et acétamide .

4. Procédé selon la revendication 1, dans le quel le solvent est choisi dans le groupe des alcools constitué du méthanol et de l'alcool isopropylique.

5. Procédé selon la revendication 1, dans le quel le solvant est un solvant organique miscible a l'eau tel que le THF.

6. Procédé selon la revendication 1, dans le quel la dite base forte est choisie dans le groupe constitué par une base organique, une base inorganique, un hydroxide de metal alcalin et un carbonate alcalin.

7. Procédé selon la revendication 6, dans le quel la dite base organique est choisie dans le groupe consistant en diéthylamine, triéthylamine et diisopropyléthylamine

8. Procédé selon la revendication 6, dans le quel le dit hydroxide de métal alcalin est choisi dans le groupe constitué par l'hydroxyde de sodium, l'hydroxyde de calcium et l'hydroxyde de potassium.

9. Procédé selon la revendication 6, dans le quel le dit carbonate alcalin est choisi dans le groupe constitué par le carbonate de sodium et le carbonate de potassium.
